# EUROPEAN PATENT SPECIFICATION

(11) **EP 2 323 676 B1**
(45) Date of publication and mention of the grant of the patent: **15.07.2015**
(21) Application number: 08874801.7
(22) Date of filing: 30.09.2008
(51) Int. Cl.: A61K 36/48, A61K 38/00, C07K 1/14, A61P 3/04, A61P 3/06, A61P 3/00, A23J 1/14, A23K 1/16, A23L 1/305, A61K 38/02

(54) **PROTEIN BASED PRODUCT FROM FENUGREEK SEEDS THAT REGULATES DYSLIPIDEMIA AND OBESITY, AND A PROCESS FOR THE PREPARATION THEREOF**
FENCHELSAMEN-PRODUKT AUF PROTEINBASIS ZUR REGULIERUNG VON DYSLIPIDÄMIE UND ADIPOSITAS UND VERFAHREN ZU SEINER HERSTELLUNG
PRODUIT À BASE DE PROTÉINE À PARTIR DE GRAINES DE FENUGREC SÉCHÉES QUI RÉGULE LA DYSLIPIDÉMIE ET L'OBÉSITÉ, ET PROCÉDÉ POUR LA PRÉPARATION DE CELUI-CI

(30) Priority: 25.06.2008 IN DE15212008
(43) Date of publication of application: 25.05.2011
(73) Proprietor: Department of Biotechnology (DBT), New Delhi 110 003 (IN); National Center for Cell Sciences, Pune 411 007 MAH (IN)
(72) Inventor: PANDEY, Vimal, Pune - 411 007 Maharashtra (IN); VIJAYAKUMAR, Malepilli, Vavachan, Pune - 411 007 Maharashtra (IN); BHAT, Manoj, Kumar, Pune 411 007 (IN)
(74) Representative: Radkov, Stoyan Atanassov
(86) International application number: PCT/IN2008/000877
(87) International publication number: WO 2009/157013

(56) References cited:
- WO-A1-99/25370
- WO-A1-2007/138609
- WO-A1-2007/138609
- US-A1- 2005 008 716
- US-A1- 2005 153 001
- VIJAYAKUMAR MALEPPILLIL VAVACHAN ET AL: "The hypoglycaemic activity of fenugreek seed extract is mediated through the stimulation of an insulin signalling pathway", BRITISH JOURNAL OF PHARMACOLOGY, NATURE PUBLISHING GROUP, BASINGSTOKE, HANTS; GB, vol. 146, no. 1, 1 September 2005 (2005-09-01), pages 41-48, XP002568317, ISSN: 0007-1188, DOI: 10.1038/SJ.BJP.0706312 [retrieved on 2005-06-27]
- SHARMA R D ET AL: "EFFECT OF FENUGREEK SEEDS ON BLOOD GLUCOSE AND SERUM LIPIDS IN TYPE I DIABETES", EUROPEAN JOURNAL OF CLINICAL NUTRITION, XX, XX, vol. 44, no. 4, 1 January 1990 (1990-01-01), pages 301-306, XP009047500,
- RAJU J ET AL: "Alleviation of hepatic steatosis accompanied by modulation of plasma and liver TNF-alpha levels by Trigonella foenum graecum (fenugreek) seeds in Zucker obese (fa/fa) rats", INTERNATIONAL JOURNAL OF OBESITY, NEWMAN PUBLISHING, LONDON, GB, vol. 30, no. 8, 1 August 2006 (2006-08-01) , pages 1298-1307, XP009156917, ISSN: 0307-0565, DOI: 10.1038/SJ.IJO.0803254 [retrieved on 2006-02-14]
- GUPTA A ET AL: "Effect of Trigonella foenum-graecum (fenugreek) seeds on glycaemic control and insulin resistance in type 2 diabetes mellitus: a double blind placebo controlled study.", THE JOURNAL OF THE ASSOCIATION OF PHYSICIANS OF INDIA NOV 2001 LNKD- PUBMED:11868855, vol. 49, November 2001 (2001-11), pages 1057-1061, XP009156916, ISSN: 0004-5772
- OSMAN M K ET AL: "Biochemical studies of some non-conventional sources of protein. Part 5. Extraction and characterization of protein from fenugreek seed (Trigonella foenum L.)", NAHRUNG - FOOD, VCH VERLAGSGESELLSCHAFT, WEINHEIM, XX, vol. 35, no. 3, 1 January 1991 (1991-01-01), pages 303-308, XP009156907, ISSN: 0027-769X, DOI: 10.1002/FOOD.19910350309 [retrieved on 2006-10-19]
- GURIB-FAKIM, A.: 'Medicinal plants: traditions of yesterday and drugs of tomorrow.' MOLECULAR ASPECTS OF MEDICINE vol. 27, no. 1, 2006, page 1, XP025089073

## Description

### Field of Invention

This invention relates to a protein based product, a novel preparation from a protein rich fraction of Fenugreek Seeds (TEFS) that reduces accumulation of fat and enhances LDL (low-density lipoprotein) uptake and, a process for the preparation thereof with potential application in the management of dyslipidemia and obesity.

### Background of Invention

Dyslipidemia and obesity are major health problems and significant risk factor for many serious diseases including cardiac diseases, cancer, arthritis and diabetes. Dyslipidemia is a disorder of lipoprotein metabolism, including lipoprotein overproduction or deficiency. Dyslipidemia may be manifested by elevation of total cholesterol, the "bad" LDL cholesterol (LDLc) and the triglyceride (TG) concentrations, and a decrease in the "good" high-density lipoprotein (HDL) cholesterol concentration in the blood. Obesity is a worldwide epidemic characterized by excess adipose tissue that contributes to numerous chronic diseases and early mortality. The adverse health consequences associated with obesity include cardiovascular diseases, stroke, type-2 diabetes mellitus, hypertension, dyslipidemia, cancers of breast, endometrium, prostrate and colon, osteoarthritis, respiratory problems, asthma and sleep apnea. Dyslipidemia and obesity occur as a result of combination of genetic, environmental and psychological factors and are characterized by an imbalance in energy metabolism resulting from excessive food intake coupled with decreased energy expenditure. This imbalance interferes with energy homeostasis, a regulatory process involving hormones such as insulin and leptin that are circulated at levels in proportion to body fat content and act on the hypothalamus to reduce food intake and fat stores. The disease is closely associated with insulin resistance together with elevated levels of TG, low HDL cholesterol (HDLc) and high LDLc levels. The triglyceride (TG) level is one of several lipid parameters that can aid prediction of coronary heart disease (CHD) risk. An elevated plasma TG level is strongly associated with an increased risk of CHD. Hypertriglyceridemia, the second most common dyslipidemic abnormality in hypertensive subjects after increased LDLc, is defined by the National Cholesterol Education Programme (NCEP) as a fasting TG level of > 2.26 mmol/l (> 200 mg/dl) and is recognized as a primary indicator for treatment in type IIb dyslipidemia.

Fat loaded adipose tissue contains TG, steroids and cholesterol. These serve as a site for TG storage and free fatty acid (FFA)/glycerol release in response to changing energy demands. In addition, adipose tissues secrete factors that regulate appetite, body energy expenditure (thermogenesis) and insulin sensitivity. Both "obesity avoidance" and "starvation avoidance" responses are regulated by homeostasis of energy stores with the signals from these systems being interpreted by the hypothalamus to determine the appropriate response. Unfortunately, the physiological mechanisms that suppress food intake and increase energy expenditure are not sufficiently robust to combat the environmental and psychological pressures that favor dyslipidemia and obesity.

### Prior Art

Current treatment of dyslipidemia consists of Statin based drugs (atorvastatin, simvastatin), ciprofibrate (phenofibrate, gemfibrozil), resins, nicotinic acids, vytorin and zetia. Management of obesity includes various approved drugs viz. orlistat, sibutramine, ephedrine, caffeine for long term use and benzphetamine, phendimetrazine, phentamine, chitosan, conjugated linoleic acid for short term use with significant side effects. Orlistat acts by inhibiting pancreatic lipase and sibutramine a 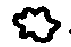 -phenethylamine that selectively inhibits the reuptake of noradrenaline, serotonin and to a less extent dopamine resulting in reduced intake of food. Ephedrine and caffeine are thermogenic drugs enhancing oxidation of fatty acids and hence classified as a nutrient partitioning agent. Due to the side effects of limited number of drugs and accompanying burden of economy, medicinal plant products have been projected as an alternative to the current crisis to manage dyslipidemia, obesity and metabolic disorders associated with these diseases in 21^{st} century. Fenugreek (*Trigonella foenum graecum*), an annual medicinal plant of Fabaceae family is well documented for pharmacological properties. Fenugreek Seeds and its extract are rich in proteins and are widely studied for their assumed medicinal properties in the treatment and prevention of several human diseases including metabolic disorders. Clinical studies concerned with the application of Fenugreek Seeds in diabetes and control of dyslipidemia/ obesity unarguably support this hypothesis. Fenugreek Seeds extract is reported to cure hyperlipidemia. However, a novel preparation from Fenugreek Seeds Proteins which has been subjected to systematic study for the efficacy in the prevention and management of dyslipidemia and obesity related complications do not exist. Moreover, no attempt has been made to unravel the mechanism of action at molecular level. Therefore, this invention was designed to examine the ability of this novel protein based product from Fenugreek Seeds to prevent some of the complications and biochemical dysfunctions associated with dyslipidemia and obesity both *in vivo* in C57BL/6J mice fed on high fat diet as well as *in vitro* in mammalian 3T3-L1 and HepG2 cells.

### Drawbacks of Prior art

Patients with dyslipidemia, especially hypertriglyceridemia have been shown to respond to dietary control and to the use of lipid lowering drugs such as 3-hydroxy-3-methylglutaryl-Coenzyme A (HMG CoA) reductase inhibitors (known as statins), fibrates and nicotinic acids to a limited extent only. However, recent retrospective real-life clinical studies show that only 38% of patients receiving some form of lipid-lowering therapy achieved NCEP-defined LDLc target levels, demonstrating the need for the use of more aggressive or alternative treatment. In hypertriglyceridemic patients, the newer statins, cerivastatin and atorvastatin, have shown limited efficacy in reducing TG. A recent study published in New England Journal of Medicine reports that Vytorin and Zetia failed to improve heart disease. Orlistat is a hydrogenated derivative of a bacterial lipase inhibitor that acts locally to block the absorption of fat by inhibiting lipases present in the gastrointestinal tract. However, its side effects include reduction in the absorption of some important liposoluble vitamins that capsizes the balance of one's nutritional requirements, oily spotting, faecal urgency and increased defecation. Sibutramine inhibits the uptake of serotonin and noradrenaline and therefore decreases food intake. Their side effects include a change in heart rate arid blood pressure, dry mouth, insomnia, and asthenia. Ephedrine and caffeine increase heart rate and a sense of palpitation in some patients. Therefore, there is a need for more efficient, safe and economic drugs.

Though hypolipidemic effects of Fenugreek (leaves/seeds) have been attributed to several factors and mechanisms, its active principles have been only partially purified viz., saponin, which accounts for the hypocholesterolemic activity in part. Fenugreek Seeds when supplemented along with food significantly reduce serum cholesterol and TG in animals and humans. However, considering the pungent odor and bitter taste of Fenugreek Seeds, the dose (25-50 gram per day) used in those studies may not be clinically feasible. Also, many hypolipidemic compounds isolated from plants are small molecules such as alkaloids, flavanoids, glycosides, steroids, amino acids or minerals that are not suitable for pharmaceutical drug development. Additionally, medicinal plant extracts used for treating dyslipidemia and obesity might contain number of components that together contribute to over-all effectiveness. Not many attempts have been made to utilize the health benefits of proteins from plant sources especially from Fenugreek.

The whole grain of Fenugreek Seeds per 100 g of edible portion contain 369 calories, 7.8% moisture, 28.2 g protein, 5.9 g fat, 54.5 g total carbohydrate, 8 g fiber, 3.6 g ash. Though proteins are known to possess health benefits, proteins from Fenugreek Seeds were never subjected for the assessment of hypolipidemic activity *in vitro* as well as *in vivo*. In this invention, extract of Fenugreek Seeds was ammonium sulphate precipitated to obtain total soluble proteins, dialyzed for 24 h with an objective to eliminate small molecules. This dialyzed protein rich fraction was heat treated to obtain a thermostable preparation. The hypolipidemic potential of this protein preparation (TEFS) was investigated *in vivo* in mice supplemented with high fat diet (HFD). TEFS significantly improved serum chemistry profile in hyperlipidemic mice by effectively lowering TG, total cholesterol and LDLc. Moreover, this preparation also prevented weight gain in normal mice fed on high fat diet. In the present study the mechanisms by which TEFS attenuates hyperlipidemia were further investigated in *in vitro* models for adipocytes differentiation and fat accumulation.

This invention presents a novel protein based product, from Fenugreek Seeds (TEFS) that decreases accumulation of fat in adipocytes, decreases triglyceride and cholesterol synthesis in liver cells and upregulates LDL receptor (LDLR) in liver cells. The product derived from protein rich fraction of Fenugreek Seeds possesses hypolipidemic activity and has potential to ameliorate complications associated with dyslipidemia and obesity.

### Objects of the Invention

The object of this invention is to derive a protein based product from Fenugreek Seeds for the management of dyslipidemia and obesity.

Other object is to define a protocol for a preparation which is devoid of small molecules.

Another object is to develop a protocol for the preparation of protein based product from Fenugreek Seeds that inhibits fat accumulation in adipocytes and, TG and cholesterol synthesis in liver.

Yet another object is to develop a protocol for preparation of protein based product from Fenugreek Seeds that enhances LDL uptake and hence improves cholesterol homeostasis.

Yet another object is to develop a human equivalent dose of protein based product from Fenugreek Seeds.

Further, object is to develop the protein based product from Fenugreek Seeds whose cumulative effect of the properties would promote body weight reduction and re-establish lipid homeostasis.

Another object of this invention is to give a scientific validation for the hypolipidemic and body weight reducing activity using *in vitro* models.

### Statement of Invention

This invention relates to a Protein based product from Fenugreek Seeds (TEFS), for example 0.1 to 15 mg/ml, preferably 0.5 to 1.5 mg/ml protein, comprising: Grinding of dried Fenugreek Seeds that may or may not be germinated; suspending ground fine powder of the Fenugreek Seeds in PBS, pH 7.4 containing 0.1 mM PMSF and protease inhibitor cocktail under chilled conditions; filtering the extract through three layered cheese cloth, centrifuging; treating clear supernatant with 25 to 75 gms/litre activated charcoal powder; extracting or precipitating protein with 20 to 80% ammonium sulphate for 1-12 hours or by any other protein precipitating agent; removing the precipitate by centrifugation, re-dissolving in PBS and dialyzing against PBS at 4 to 21°C with atleast three buffer changes; heat treating the dialyzed protein rich extract at 50 to 95°C for 15 to 60 min, preferably at 75-90°C for 30 min; removing the precipitated protein by centrifugation and retrieving supernatant as final product; the said final product has a protein concentration of 0.1-15 mg/ml, preferably 1-1.5 mg/ml; the said product, a novel protein based product from Fenugreek Seeds is useful in the management of dyslipidemia and obesity;

### Brief description of accompanying drawings

Figure 1 shows: Fingerprint of a novel protein based product from Fenugreek Seeds (TEFS). Five to 50 µg of protein based product from Fenugreek Seeds was resolved on an 8-10% gel and stained with Coomassie brilliant blue or silver stain, or resolved in columns attached to Biologic DuoFlow high-resolution chromatographic system.
Figure 2 demonstrates: Treatment of protein based product from Fenugreek Seeds inhibits fat accumulation in differentiating 3T3-L1 cells *in vitro*. 3T3-L1 cells were treated with different concentration of protein based product from Fenugreek Seeds from day 0 to 11^{th} day of differentiation in Petri plates.
Figure 3 shows: Representative picture of unstained and Oil Red O stained preadipocytes and Oil Red O stained adipocytes treated without or with different concentrations of protein based product from Fenugreek Seeds.
Figure 4 shows: Effect of protein based product from Fenugreek Seeds on TG and cholesterol levels in differentiating and differentiated cells.
Figure 5 demonstrates: Silver staining analysis of protein expression in protein based product from Fenugreek Seeds treated 3T3-L1 cells. Cells were lysed and equal amount of whole cell lysate protein was resolved by SDS-PAGE followed by silver staining.
Figure 6 shows: Western blot analysis of 3T3-L1 cells treated with different concentrations of protein based product from Fenugreek Seeds from day 0 to 11^{th} day of differentiation.
Figure 7 illustrates: Effect of protein based product from Fenugreek Seeds on total triglyceride and cholesterol levels in human hepatoma cells. HepG2 cells cultured in normal media were treated with 50 µg/ml TEFS for 18 h.
Figure 8 shows: Effect of protein based product from Fenugreek Seeds on LDLR expression in human hepatoma cells cultured in cholesterol enriched media.
Figure 9 demonstrates: After 15-day treatment with protein based product from Fenugreek Seeds at a dose of 1.5 mg/kg /d and 15 mg/kg/d decreased body weight, TG, total cholesterol and LDLc respectively in comparison to high fat diet fed animals administered control solution

### Detailed description of invention

The present invention provides a method for preparation of a protein based product from a protein rich fraction of Fenugreek Seeds and methods of using the same to promote loss of adipose fat and liver lipids for the management of dyslipidemia and obesity. The preparation can further be used to improve serum chemistry or metabolic parameters such as blood triglyceride levels, cholesterol levels and LDL cholesterol levels in addition to promoting weight loss, in subjects. Proteins present in the extract have health benefits in a subject. Additionally, the report provides scientific basis for its usage by documenting mechanism of its action.

In this presentation inventors have defined a novel protocol for preparation of extract of Fenugreek Seeds, which exhibits hypolipidemic activity and promotes body weight reduction. This extract has potential therapeutic applications in patients for management of dyslipidemia and obesity.

This invention provides a novel protein based product from Fenugreek Seeds for the management of dyslipidemia and obesity, and a protocol comprising:
I) grinding of dried Fenugreek Seeds that may or may not be germinated;
II) suspending ground fine powder of the seeds in PBS, pH 7.4 containing 0.1 mM PMSF and protease inhibitor cocktail;
III) filtering the extract through three layered cheese cloth, centrifuging;
IV) treating clear supernatant with 25 to 75 gms/litre activated charcoal powder;
VI) extracting or precipitating protein with 20 to 80% ammonium sulphate for 1-12 hours;
VII) removing the precipitate by centrifugation, re-dissolving in PBS and dialyzing against PBS at 4 to 21°C with at least three buffer changes;
VIII) heat treating the dialyzed protein rich extract at 50 to 95°C for 15 to 60 min;
IX) removing the precipitated protein by centrifugation and retrieving final product as supernatant;
X) the final product has a protein concentration 0.1-15 mg/ml, preferably 1-1.5 mg/ml;
XI) the said product decreases lipids in adipocytes and liver cells of mammals;
XII) the said product enhances LDL cholesterol uptake in human liver cells;
XIII) the said product is capable of improving serum chemistry profile and lipid homeostasis in mammals;
XIV) the said product is capable of decreasing body weight in mammals;
XV) the said product, a novel protein based product from Fenugreek is useful in the management of dyslipidemia and obesity.

In the present invention surface sterilized Fenugreek Seeds were extracted in PBS containing protease inhibitors. The aqueous extract of Fenugreek Seeds was ammonium sulphate precipitated to extract soluble proteins, dialyzed against PBS to remove salt and small molecules and heat treated to obtain protein based product from Fenugreek Seeds. The hypolipidemic potential of this product was investigated *in vivo.* Protein based product from Fenugreek Seeds significantly improved serum chemistry profile viz. TG, total cholesterol and LDLc with significant reduction in the body weight of mice fed a high fat diet. In the present study the mechanisms by which TEFS attenuates dyslipidemia and body weight were further investigated *in vitro* using differentiating as well as differentiated 3T3-L1 cells as a model for adipocytes and human hepatoma cell line, HepG2 cells as a model for liver cells.

Inventors obtained PVDF membranes from Amersham Biosciences (Piscataway, NJ), DEX, IBMX, insulin (bovine pancreas), nonidet P-40 (NP-40), and other fine chemicals were purchased from Sigma (St. Louis, MO). DMEM, fetal bovine serum (FBS), were purchased from Invitrogen

(Carlsbad, CA). All electrophoresis reagents, molecular weight markers and peroxidases-conjugated secondary antibodies were obtained from Bio-Rad (Hercules, CA). Protease inhibitor cocktail (Tm complete) was purchased from Roche Diagnostics GmbH (Mannheim, Germany) and dialysis membrane was from Thomas Scientific (Philadelphia, PA). Super signal reagent for enhanced chemiluminescence and coomassie plus protein assay reagent were obtained from Pierce (Rockford, IL). Polyclonal antibodies against PPAR-γ, SREBP-1, ACC, aP2, GAPDH, C/EBP-α GLUT4, GLUT1, β-tubulin, LDLR and β-actin were procured from Santa Cruz Biotechnology (Santa Cruz, CA). Triglyceride estimation kit was obtained from Spinreact (Girona, Spain), and Cholesterol estimation kit was obtained from Ensure Biotech. (Hyderabad, India).

### Preparation of protein based product from Fenugreek Seeds (TEFS)

Dried, viable, and fresh batches of Fenugreek Seeds were obtained from a commercial source (Mona spices Co. Ltd., Pune, India). Surface sterilized seeds were ground to a fine powder in a mixer under chilled conditions and were suspended in PBS (pH 7.4) containing 0.1 mM PMSF and protease inhibitor cocktail. The extract was filtered through three-layered cheesecloth, centrifuged and the clear supernatant was treated with activated charcoal powder 25-75 gms/litre. Proteins from clarified extract were precipitated with 20% to 80% ammonium sulphate for 1-12 h. Precipitate was removed by centrifugation, redissolved in PBS and dialyzed against PBS with at least three buffer changes at 4° to 21°C. Dialyzed protein rich solution was then heat treated at 50 to 100°C preferably 75-90°C, for 15 to 60 min, preferably 30 min. Precipitated proteins were removed by centrifugation and clear supernatant was obtained. This clear supernatant, a protein based product from Fenugreek Seeds was termed as TEFS. Extract thus prepared has a protein concentration of 0.5- 1.5 mg/ml. This was aliquoted, stored at - 70°C for long term and was used for all further experiments.

### Electrophoretic Fingerprinting and Analysis of TEFS

Five to 50 µg of TEFS was resolved on an 8-10% polyacrylamide gel and stained with Coomassie brilliant blue or silver stain. Molecular weight of the protein bands present in the extract was compared with the molecular weight of the standard protein markers.

### Chromatographic Analysis of TEFS

Two to 5 mg TEFS was analyzed at 280 nm in Biologic DuoFlow high resolution chromatographic system (BioRad, Hercules, CA, USA), using Bio-Sil SEC HPLC column (300X7.8 mm, flow rate 1 ml/min, isocratic, Buffer 50 mM ammonium bicarbonate, pH 7.5) or Uno Q1 ion exchange column (7X35 mm, flow rate 1ml/min, linear gradient of sodium chloride in 25 mM Tris-HCl, pH 8.0 for 45 min).

### Cell Culture and Induction of Differentiation

3T3-L1 pre-adipocytes and HepG2 cells were obtained from ATCC, Virginia, USA and maintained in DMEM containing 10% FBS. 3T3-L1 cells were grown to 80-90% confluence in 24 well plates or 60 mm coated Petri dishes. Differentiation was induced by incubating the cells in DMEM media containing 10% FBS along with a hormone mixture consisting of 250 nM DEX, 0.5 mM IBMX and 100 nM insulin on day zero. After 48 h, the medium was replaced with DMEM supplemented with 10% FBS and 100 nM insulin. After additional 48 h, insulin was withdrawn, and the medium was changed every second day. To determine the role of TEFS in pre-adipocyte differentiation, extract was added as a supplement from day zero or day 5 and also during every media change. The differentiated cells 9-12 days after the induction of differentiation were washed thrice with ice cold PBS and lysed in 100 ml of lysis buffer (20 mM HEPES, pH 7.4, containing 1% Triton X-100, 2 mM EDTA, 100 mM NaF, 10 mM sodium pyrophosphate, 1mM PMSF, 1 mM TPCK and protease inhibitor cocktail tablet per 1X10⁶ cells on ice. Lysates were passed through 261/2 gauge syringe and centrifuged at 15000 rpm for 20 min. Equal amount of protein samples were resolved on 10-12% SDS using standard protocol or transferred onto PVDF membrane. The membranes were probed with antibodies against PPAR-γ, SREBP-1, ACC, aP2, GAPDH, C/EBP-α, GLUT4, GLUT1, β-tubulin and β-actin. Alternatively, cells were lysed with 2% TritonX-100 and total TG and cholesterol were estimated using respective kits.

### Oil Red O Staining

Cells were stained by 0.2% Oil Red O to visualize directly oil droplets accumulated in the differentiated cells. In brief, cells were washed with cold PBS and fixed with 3% paraformaldehyde. After two washes with PBS, Oil Red O was added for 30 min, followed by washing with PBS. For each well or plate, three images were taken.

### Measurement of Total Triglyceride and Cholesterol Level in HepG2 Cells

Cells were incubated with 50 µg/ml TEFS for 18 h. Cells were washed with chilled PBS and lysed with 2% triton X-100. Total TG and cholesterol were estimated using respective kits.

### Immunofluorescence Microscopy

HepG2 cells were cultured in 12 well slides (ICN, Ohio, USA). On next day cells were washed with PBS, incubated in DMEM containing 0.5% LPDS supplemented with sterols (10 µg/ml cholesterol+1 µg/ml 25-hydroxycholesterol) and oncostatin M, or TEFS for 24 h. Cells were fixed with 3% parafomaldehyde for 15 min, washed twice with PBS and incubated with 0.1% glycine for 5 min. Following blocking with 1% BSA and 5% FBS in PBS, the cells were probed with anti-LDLR antibody at 1:50 dilution. The primary antibodies were visualized with FITC conjugated goat anti-rabbit 1gG (1:100 dilution). Fluorescence signals were evaluated with a Zeiss confocal laser scan microscope (Zeiss, Oberkochen, Germany).

### Animal Experiments

Thirty five, male, C57BL/6J mice 10 weeks old weighing ∼20 g were used in the experiments. Before treatment 30 animals were acclimatized for fifteen days to high fat diet (normal feed supplemented with 400 g ground nut and 200 g dried coconut per kg bodyweight of mice) and continued throughout the experiment. Next day (day zero) animals were divided into 3 groups (n=10). Animals were given TEFS orally (1.5 mg/kg/d or 15 mg/kg/d). Oral gavage was used to administer TEFS or vehicle. The diet control group comprised of five mice fed on a normal diet without drug treatment. Body weight, TG, total serum cholesterol, and LDLc were measured after 6 h fasting before (day 0), during and after the course of treatment.

### Statistics

Results are expressed as mean ± SD. All statistical analyses were done using student's t test. P value < 0.05 is considered significant in comparison to respective control groups.

### Pictorial representation of invention

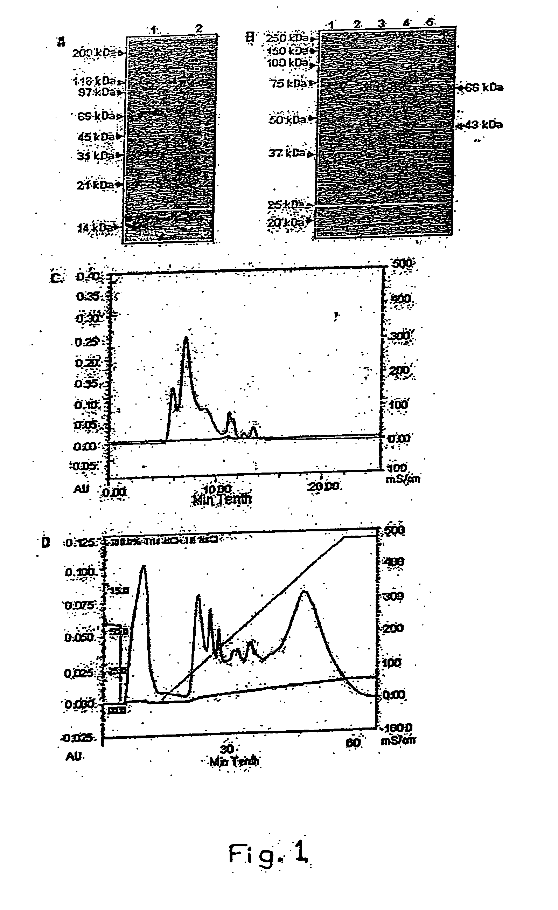

Fingerprint of protein based product from Fenugreek Seeds (TEFS). Five to 50 µg of TEFS was resolved on an 8-10% polyacrylamide gel and stained with Coomassie brilliant blue or silver stain, or resolved in columns attached to Biologic DuoFlow high resolution chromatographic system. A Silver stained gel. Lane 1 standard protein marker and lane 2 TEFS, 5 µg. B. Coomassie stained gel. Lane 1 Standard molecular weight protein marker, lane 2-4, 5, 25 and 50 µg TEFS, and lane 5 standard BSA (66 kDa) and ovalbumin (43 kDa) 1 µg each. C. Protein peak profile of TEFS resolved on Bio-Sil SEC HPLC column. D. Protein peak profile of TEFS resolved on Uno Q1 ion exchange column (Fig.1)

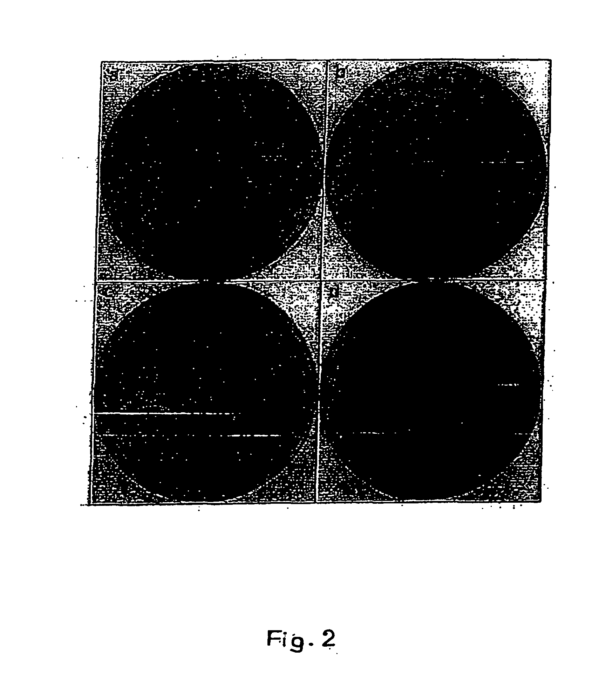

TEFS treatment inhibits fat accumulation in differentiating 3T3-L 1 cells *in vitro.* 3T3-L1 cells were treated with different concentration of TEFS from day 0 to 11^{th} day of differentiation in petri plates. On 11^{th} day, cells were fixed and stained with Oil-Red O that is specific for TG. (a) Completely differentiated 3T3-L1 cells, (b), (c) and (d) 3T3-L1 cells treated with TEFS 0.5, 5 and 50 µg/ml respectively during differentiation. TEFS treatment decreased the TG accumulation as indicated by Oil Red O staining (Fig. 2).

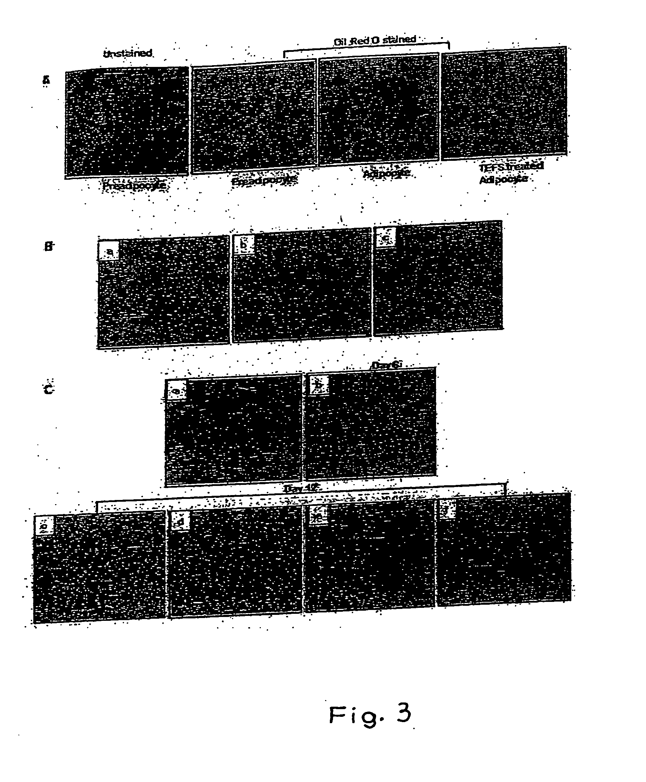

Effect of TEFS on accumulation of oil droplets in differentiating and differentiated cells. A. Representative pictures of unstained and Oil Red O stained preadipocytes and Oil Red O stained adipocytes treated without or with TEFS B. Photographs of Oil Red O stained 3T3-L1 adipocytes treated with different concentrations of TEFS (a) completely differentiated 3T3-L 1 cells; (b) and (c), 3T3-L1 cells treated with TEFS 5 and 50µg/ml respectively. TEFS treatment decreased the TG accumulation as indicated by Oil Red O staining. C. 3T3-L1 cells were differentiated for 6 days followed by treatment with TEFS for another 6 days. On day 12 cells were fixed and Oil Red O stained. (a) Undifferentiated 3T3-L1 preadipocytes, (b) 3T3-L1 cells differentiated for 6 days (c) completely differentiated 3T3-L1 cells; (d) (e) and (f) differentiated 3T3-L1 cells treated with TEFS 0.5, 5 and 50µg/ml respectively. TEFS treatment decreased the TG accumulation as indicated by Oil Red O staining (Fig. 3).

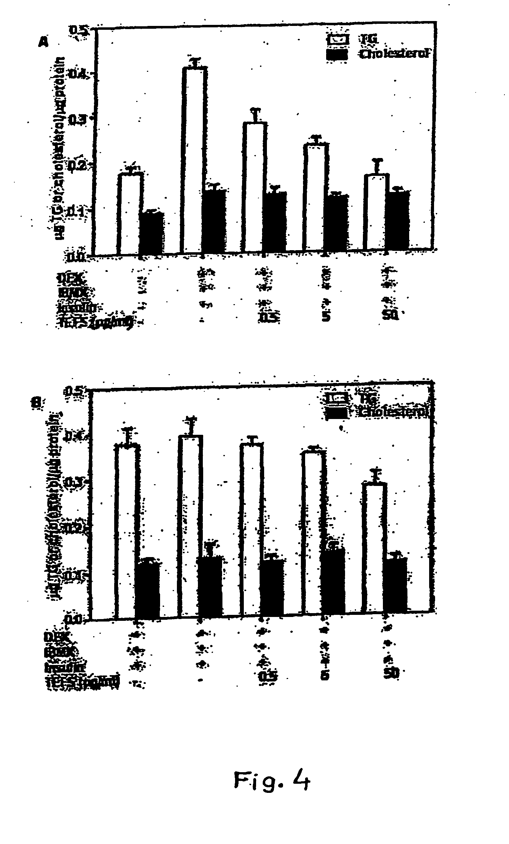

Effect of TEFS on TG and cholesterol levels in differentiating and differentiated cells. A. 3T3-L1 cells treated with TEFS from day 0 to 11^{th} day of differentiation. B. 3T3-L1 cells were differentiated for 6 days followed by treatment with TEFS for another 6 days. Cells were lysed with 2% TritonX-100 for TG and cholesterol estimation (Fig. 4).

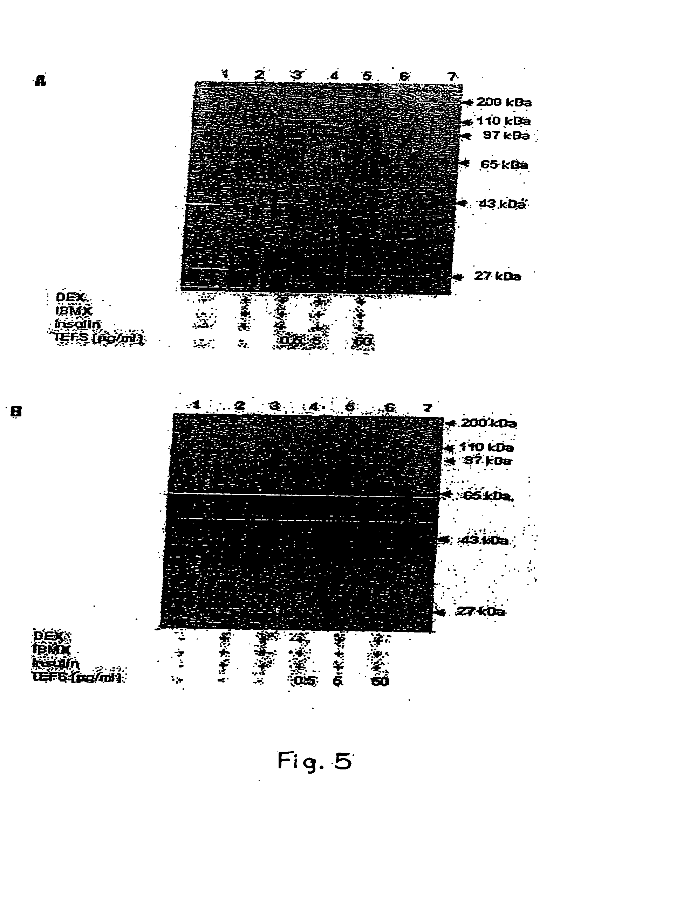

Silver staining analysis of protein expression in TEFS treated 3T3-L1 cells. Cells were lysed and equal amount of whole cell lysate protein was resolved by SDS-PAGE followed by silver staining. A. 3T3-L1 cells treated with TEFS from day 0 to 11^{th} day of differentiation. Lane 1 represents preadipocytes and lane 7 is standard protein molecular weight markers. B. 3T3-L1 cells were differentiated for 6 days followed by treatment with TEFS for another 6 days. Lane 1 preadipocytes, lane 3 is cells differentiated for 6 days and lane 7 is standard protein molecular weight markers. TEFS treatment altered the expression profile of proteins corresponding to arrow marks within the gel (Fig. 5).

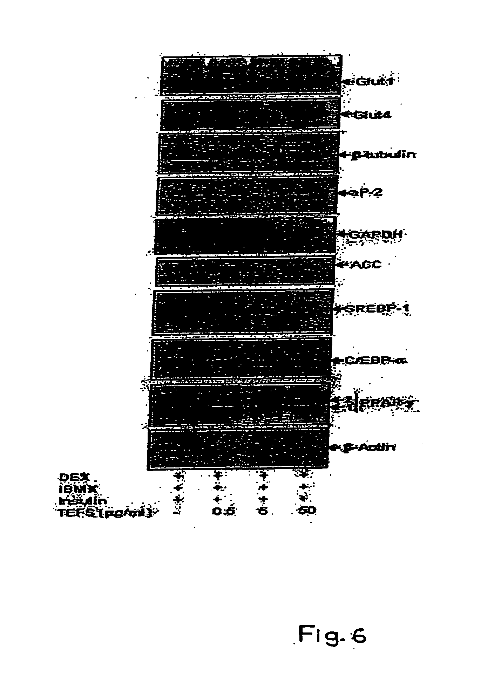

3T3-L1 cells treated with different concentrations of TEFS from day 0 to 11^{th} day of differentiation. Cells were lysed and equal amount of protein was resolved by SDS-PAGE, transferred to PVDF membrane followed by immunoblotting analysis of desired antibodies. Differentiation of 3T3-L1 cells led to the increased expression of PPAR-γ, C/EBP-α, ACC, SREBP, GAPDH that are implicated in fat accumulation and adipogenesis. TEFS treatment in these cells decreased the expression of these proteins implicated in fat accumulation in a dose dependent manner (Fig. 6).

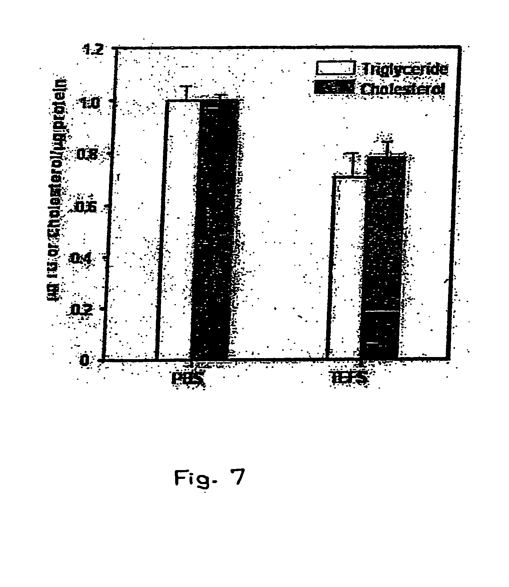

Effect of TEFS on total triglyceride and cholesterol levels in human hepatoma cells. HepG2 cells cultured in normal media were treated with 50 µg/ml TEFS for 18 h. Cells were lysed and total TG and cholesterol were estimated. TEFS significantly decreased total triglyceride and cholesterol as compared to PBS treated control cells (Fig. 7).

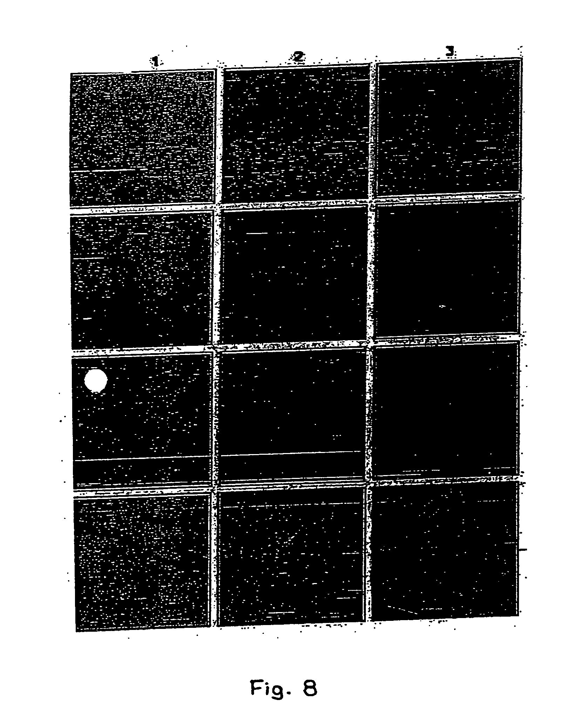

Effect of TEFS on LDLR expression in human hepatoma cells cultured in cholesterol enriched media. A. HepG2 cells cultured in normal media. In B, C and D panels, HepG2 cells cultured in cholesterol enriched media treated with PBS, Oncostatin M or TEFS respectively. Cells were probed with LDLR antibody. Immunostained cells were analyzed with confocal microscopy. Lane 1, phase contrast image, lane 2 images of LDLR immunostaining and lane 3 superimposed images of LDLR staining and nuclear staining. TEFS treatment enhanced the LDLR expression on the membranes of HepG2 cells as compared to PBS treatment. Effects of TEFS on LDLR expression was comparable to that mediated by Oncostatin M (Fig. 8).

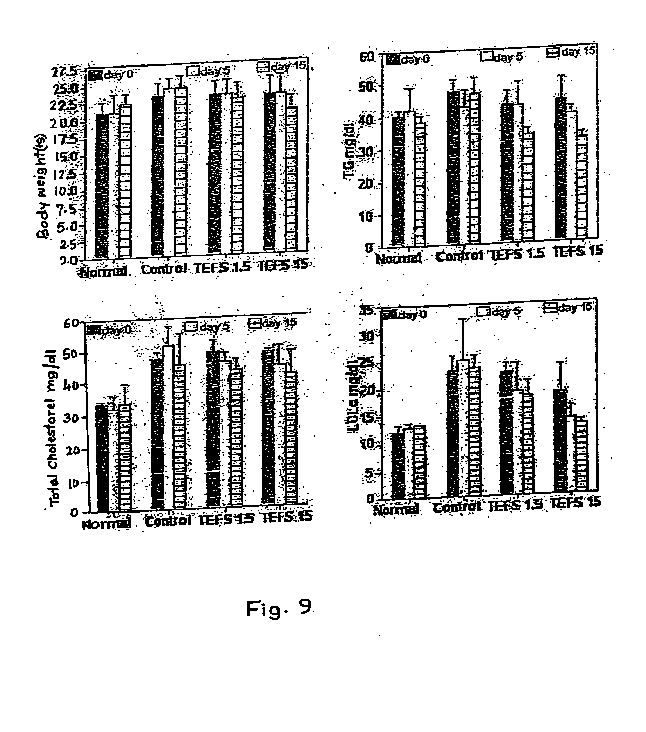

Mice were acclimatized for fifteen days to high fat diet. Next day (day zero) animals were divided into 3 groups (n=10) and they were continued to be fed on high fat diet throughout experiment. Mice were administered orally either with control solution or TEFS (1.5 mg/kg/d and 15 mg/kg/d doses). Five mice provided with normal feed were used as normal control animals. Body weight, serum cholesterol, triglyceride and LDLc were measured after 6 h fasting before (day 0), during and after the course of treatment. Fifteen day treatment with TEFS at a dose of 1.5 mg/kg/d and 15 mg/kg/d decreased body weight, TG, total cholesterol and LDLc respectively in comparison to high fat diet fed animals administered control solution (Fig. 9).

### Fingerprinting and Analysis of TEFS

The protein based product from Fenugreek Seeds (TEFS) contained 0.1 to 15 mg/ml protein, preferably 0.5-1.5 mg/ml protein as per the protein estimation using coomassie plus protein assay reagent. Electophoretic separation of TEFS followed by coomassie blue or silver staining suggested that TEFS contains proteins of molecular weight <65 kDa (Fig. 1, A and B). Twelve and ten protein bands were visible by silver and coomassie staining respectively. Chromatographic separation of proteins present in TEFS using Bio-Sil SEC HPLC column reveals 7 partially resolved peaks at 280 nm (Fig. 1C). Using Uno Q 1 ion exchange column, we have shown that most of the proteins bind to the column at pH 8.0 and elute with a linear gradient of NaCl (0-1M) producing 7 partially resolved peaks (Fig 1D).

### TEFS Inhibited Lipid Droplet Accumulation in Differentiating as well as Differentiated Cells

Confluent 3T3-L1 pre-adipocytes upon exposure to an adipogenic hormone mixture (250 nM DEX, 0.5 mM IBMX and 100 nM insulin) transformed into fat-laden adipocytes in 6-8 days. The conversion process could be monitored by the gradual accumulation of cytoplasmic TG predominantly in the form of fat droplets compared to preceding cells, as indicated by variation in adipose cell size observed under light microscope as well as by the staining of lipid droplets using Oil Red O stain by day 5-7 (Fig. 2 and 3 A). To investigate the potential inhibitory effects of TEFS on adipocyte differentiation, various concentrations of TEFS were added to confluent 3T3-L1 cells at the outset of the differentiation program. Additionally, to test the effect of TEFS on the lipids accumulated in differentiated adipocytes we treated 6 day post differentiated adipocytes with TEFS for additional 6 days. The same concentration of TEFS was added subsequently at 48 h intervals each time and medium was replaced. Oil Red O staining and microscopic examination of TEFS treated cells cultured under optimum differentiation condition revealed a significant reduction in the accumulation of cytoplasmic fat in a dose dependent manner (Fig. 3, B and C). Cells treated with 50 µg/ml of TEFS accumulated significantly low levels of intracellular TG (∼60% less) as compared to the hormone mixture treated positive controls under differentiating conditions (Fig. 4A). Similarly, TEFS in a dose dependent manner significantly reduced intracellular TG levels in post differentiated cells of six days (Fig. 4B). To exclude the possible antiproliferative or cytotoxic effects we measured the viability of cells grown under similar conditions. TEFS did not have either antiproliferative or cytotoxic effects even at concentration above 100 µg/ml. TEFS attenuates the accumulation of intracellular TG significantly in comparison to positive control (p<0.05) in differentiating as well as differentiated cells. In addition total cell count was unaffected in treated cells irrespective of the concentration of TEFS.

### TEFS Attenuated the Expression of Adipogenic Factors

We next examined the effect of TEFS on the expression of adipogenic transcription factors. During the process of differentiation, cells express various differentiation markers including lipogenic and lipolytic enzymes, as well as transcription factors and proteins that include C/EBP-β, δ, α, PPAR-γ, SREBP, GAPDH, GLUT4 etc, which modulate the mature adipocytes phenotype. To determine if reduced lipid accumulation resulted from TEFS mediated alteration in differentiation program, the expression of proteins in 3T3-L1 cells treated with TEFS during the process of differentiation or on day 6 following initiation of differentiation, were studied by silver staining the lysates prepared from the experimental cells (Fig.5). TEFS treatment significantly altered the expression profiles of proteins as compared to the cells that were propagated in only complete differentiation media. Further analysis of expression of adipogenic factors in TEFS treated differentiating cells by immunoblotting suggested that expression of adipogenic transcription factors viz. PPAR-γ, C/EBP-α, SREBP-1, ACC (acetyl CoA carboxylase) and GAPDH were significantly inhibited in a dose dependent manner as compared to cells treated with hormone mixture only. TEFS did not alter the expression of GLUT1, GLUT4, aP-2 and β-tubulin (Fig.6).

### TEFS Suppresses Triglyceride and Cholesterol Level in Human Hepatoma Cell Line, HepG2 Cells

Enhanced TG and cholesterol levels are implicated in dyslipidemia. Treating HepG2 cells with TEFS significantly (P<0.05) reduced TG and cholesterol levels in comparison to PBS treated control cells (Fig. 7).

### TEFS Enhanced LDL Uptake via Expression of LDL Receptor

Treating human hepatoma cells, HepG2, cultured in medium containing 0.5% lipoprotein-depleted serum (LPDS) or in LPDS supplemented with sterols along with TEFS resulted in the enhanced expression of LDLR as determined by staining for antibodies to LDLR (Fig. 8). The increase in LDLR expression directly correlates to enhanced LDLR function responsible for LDL uptake.

### Effects of TEFS on Body Weight and Serum Chemistry Profile of Fasted Animals.

To verify that TEFS possesses hypolipidemic activity *in vivo*, an animal study was conducted. Before TEFS treatment, animals were fed normal diet supplemented with fat (coconut and ground nut) for fifteen days. Average body weight of mice fed on diet with fat supplement increased and also resulted in enhanced TG, total cholesterol and LDLc following 15 days (day zero) of acclimatization in comparison to mice fed only normal diet. Treatment of these hyperlipidemic animals with TEFS by oral administration for 15 days resulted in decrease of serum TG, LDLc and bodyweight in a dose and time dependent manner (Fig.9). Following the 15-days treatment with TEFS at a dose of 1.5 mg/kg/day the reduction in body weight, TG, total cholesterol and LDLc was by 2.3%, 22.4%, 11.5% and 19.6% respectively. Similarly, administration of TEFS at a dose of 15mg/kg/d decreased the body weight, TG, total cholesterol and LDLc by 9.4%, 26.6%, 14.9% and 31% respectively in comparison to levels on day zero. TEFS effect on body weight, total cholesterol and LDLc was time-dependent with significant reduction on day 5 at both doses (P<0.05).

In summary, inventors have developed a totally novel protocol by which a product can be made from protein rich fraction of Fenugreek Seeds (TEFS) with potential applications in management of dyslipidemia and obesity. Exploratory mechanistic studies indicate that TEFS decreased cellular TG and cholesterol synthesis in addition to promoting receptor mediated uptake of LDLc. The *in vitro* and *in vivo* profiles of TEFS reported here provide not only a treatment for the management of hyperlipidemia but also provides a rational platform for isolating active principles that mediate these functions.

### ABBREVIATIONS:

TEFS, protein based product from Fenugreek Seeds; TG, triglyceride; LDL, low density lipoprotein; LDLc, LDL cholesterol; LDLR, LDL receptor; HDL, high density lipoprotein; HDLc, HDLcholesterol; DEX, dexamethasone; IBMX, isobutylmethylxanthine; GLUT4, glucose transporter 4; PPAR-γ, peroxisome proliforators activated receptor-gamma; C/EBP-α, CCAAT/enhancer element binding protein alpha, SREBP-1, sterol response element binding protein-1, ACC, acetyl CoA carboxylase; PBS, phosphate-buffered saline; TBS, tris-buffered saline; DMEM, Dulbecco's modified Eagle's medium; PMSF, phenylmethylsulfonyl fluoride.

## Claims

1. A method of preparing a protein-based product from fenugreek seeds (TEFS) for use in the treatment of dyslipidemia and obesity, the method comprising:
i) grinding dried fenugreek seeds to a fine powder;
ii) suspending the ground fine powder of the fenugreek seeds in phosphate-buffered saline (PBS) at pH 7.4 containing 0.1 mM phenylmethylsulfonyl fluoride (PMSF) and a protease inhibitor mixture to yield an extract;
iii) filtering the extract through a three-layered cheese cloth, centrifuging to yield a clear supernatant;
iv) treating the clear supernatant with 25 to 75 g/litre activated charcoal powder;
v) precipitating protein with 20 to 80% ammonium sulphate for 1-12 hours;
vi) removing the precipitate by centrifugation, re-dissolving in PBS and dialyzing against PBS at 4 to 21 °C with at lea st three buffer changes;
vii) heat-treating the dialyzed protein rich extract at 50 to 95 °C for 15 to 60 min, preferably at 75-90 °C for 30 min;
viii) removing the precipitated protein by centrifugation and retrieving the supernatant as a final product; wherein
the final product has a protein concentration of 0.1-15 mg/ml, preferably 1-1.5 mg/ml; and is useful in the management of dyslipidemia and obesity.

2. A protein-based product from fenugreek seeds (TEFS) obtainable from the method of claim 1 for use in the treatment of dyslipidemia and obesity.

3. A composition comprising the protein-based product for use according to claim 2, wherein the composition provides a dose of 1.5 mg to 15 mg/kg body weight to mammals daily and a human equivalent dose of 5-100 mg/kg body weight daily, comprising one or more excipient.

4. The protein-based product for use according to claims 2 to 3, wherein the use is by oral administration to a mammal and comprises the reduction of triglyceride, total cholesterol, low-density lipoprotein (LDL) cholesterol and body weight.

5. The protein-based product for use according to claims 2 to 3, wherein the use comprises reducing accumulated fat in mammalian adipocytes.

6. The protein-based product for use according to claims 2 to 3, wherein the use comprises a decrease in adipose tissue mass.

7. The protein-based product for use according to claims 2 to 3, wherein the use comprises a decrease in triglyceride and cholesterol synthesis in human liver cells.

8. The protein-based product for use according to claims 2 to 3, wherein the use comprises an increase in high-density lipoprotein (HDL) cholesterol levels in mammals.

9. The protein-based product for use according to claims 2 to 3, wherein the use comprises maintaining LDL/HDL ratio in mammal, when administered at a dose of 1.5 mg to 15 mg/kg body weight daily

10. The protein-based product for use according to claims 2 to 3, wherein the use is by administration at a human equivalent dose of 5-100 mg/kg bodyweight daily.

11. The protein-based product for use according to claims 2 to 3, wherein the use is by administration orally in solid or liquid form with or without any excipient.

## Patentansprüche

1. Verfahren zum Herstellen eines Produkts auf Proteinbasis aus Bockshornkleesamen (TEFS) zur Verwendung bei der Behandlung von Dyslipidämie und Adipositas, wobei das Verfahren Folgendes umfasst:
i) Mahlen getrockneter Bockshornkleesamen zu einem feinen Pulver;
ii) Suspendieren des gemahlenen feinen Pulvers der Bockshornkleesamen in phosphatgepufferter Kochsalzlösung (PBS) bei pH 7,4, die 0,1 mM Phenylmethylsulfonylfluorid (PMSF) und ein Proteasehemmergemisch enthält, um einen Extrakt zu ergeben;
iii) Filtrieren des Extrakts durch ein dreilagiges Käsetuch, Zentrifugieren, um einen klaren Überstand zu ergeben;
iv) Behandeln des klaren Überstands mit 25 bis 75 g/Liter Aktivkohlepulver;
v) Ausfällen von Protein mit 20- bis 80%-igem Ammoniumsulfat für 1 - 12 Stunden;
vi) Abziehen des Ausfällungsprodukts durch Zentrifugation, erneutes Lösen in PBS und Dialysieren gegen PBS bei 4 bis 21 °C mit mindestens drei Pufferwechseln;
vii) Wärmebehandeln des dialysierten, an Protein reichen Extrakts bei 50 bis 95 °C für 15 bis 60 min., vorzugsweise bei 75 - 90 °C für 30 min.;
viii) Abziehen des ausgefällten Proteins durch Zentrifugation und Gewinnen des Überstands als ein Endprodukt; wobei
das Endprodukt eine Proteinkonzentration von 0,1 - 15 mg/ml, vorzugsweise 1 - 1,5 mg/ml hat und bei der Bewältigung von Dyslipidämie und Adipositas von Nutzen ist.

2. Produkt auf Proteinbasis aus Bockshornkleesamen (TEFS), das mit dem Verfahren nach Anspruch 1 erhältlich ist, zur Verwendung bei der Behandlung von Dyslipidämie und Adipositas.

3. Zusammensetzung, die das Produkt auf Proteinbasis zur Verwendung nach Anspruch 2 umfasst, wobei die Zusammensetzung eine Tagesdosis von 1,5 mg bis 15 mg/kg Körpergewicht an Säugetiere und eine für den Menschen entsprechende Tagesdosis von 5 - 100 mg/kg Körpergewicht bereitstellt, die einen oder mehrere Hilfsstoffe umfasst.

4. Produkt auf Proteinbasis zur Verwendung nach den Ansprüchen 2 bis 3, wobei die Verwendung durch orale Verabreichung an ein Säugetier erfolgt und die Reduzierung von Triglycerid, Gesamtcholesterin, Cholesterin mit Lipoproteinen niedriger Dichte (LDL-Cholesterin) und Körpergewicht umfasst.

5. Produkt auf Proteinbasis zur Verwendung nach den Ansprüchen 2 bis 3, wobei die Verwendung das Reduzieren von angesammeltem Fett in Adipozyten von Säugetieren umfasst.

6. Produkt auf Proteinbasis zur Verwendung nach den Ansprüchen 2 bis 3, wobei die Verwendung eine Verringerung der Fettgewebemasse umfasst.

7. Produkt auf Proteinbasis zur Verwendung nach den Ansprüchen 2 bis 3, wobei die Verwendung eine Verringerung der Triglycerid- und Cholesterinsynthese in menschlichen Leberzellen umfasst.

8. Produkt auf Proteinbasis zur Verwendung nach den Ansprüchen 2 bis 3, wobei die Verwendung eine Erhöhung der Spiegel von Cholesterin mit Lipoproteinen hoher Dichte (HDL-Cholesterin) in Säugetieren umfasst.

9. Produkt auf Proteinbasis zur Verwendung nach den Ansprüchen 2 bis 3, wobei die Verwendung das Aufrechterhalten des LDL/HDL-Verhältnisses in einem Säugetier umfasst, wenn es in einer Tagesdosis von 1,5 mg bis 15 mg/kg Körpergewicht verabreicht wird.

10. Produkt auf Proteinbasis zur Verwendung nach den Ansprüchen 2 bis 3, wobei die Verwendung durch Verabreichung in einer für den Menschen entsprechenden Tagesdosis von 5 - 100 mg/kg Körpergewicht erfolgt.

11. Produkt auf Proteinbasis zur Verwendung nach den Ansprüchen 2 bis 3, wobei die Verwendung durch orale Verabreichung in fester oder flüssiger Form mit oder ohne jeglichen Hilfsstoff erfolgt.

## Revendications

1. Procédé de préparation d'un produit à base de protéines à partir de graines de fenugrec (TEFS) pour une utilisation dans le traitement de dyslipidémies et de l'obésité, le procédé comprenant :
i) le broyage de graines de fenugrec sèches en une poudre fine ;
ii) la mise en suspension de la poudre fine de graines de fenugrec broyées dans un tampon phosphate salin (PBS) à pH 7,4 contenant 0,1 mM de fluorure de phénylméthylsulfonyle (PMSF) et un mélange d'inhibiteur de protéase pour obtenir un extrait ;
iii) la filtration de l'extrait au travers de trois épaisseurs d'étamine et sa centrifugation pour obtenir un surnageant limpide ;
iv) le traitement du surnageant limpide par 25 à 75 g/litre de poudre de charbon activé ;
v) la précipitation des protéines avec 20 à 80 % de sulfate d'ammonium pendant 1-12 heures ;
vi) l'élimination du précipité par centrifugation avant redissolution dans un PBS et dialyse contre le PBS à 4-21 °C avec au moins trois changements de tampon ;
vii) le traitement thermique de l'extrait dialyse riche en protéines à 50-95 °C pendant 15 à 60 min, de préférence à 75-90 °C pendant 30 min ;
viii) l'élimination des protéines précipitées par centrifugation et la récupération du surnageant en tant que produit final ; dans lequel
le produit final a une concentration en protéines qui est de 0,1-15 mg/ml, de préférence de 1-1,5 mg/ml ; et il est utile dans la prise en charge de dyslipidémies et de l'obésité.

2. Produit à base de protéines dérivées de graines de fenugrec (TEFS) qui peut être obtenu par le procédé de la revendication 1 pour une utilisation dans le traitement de dyslipidémies et de l'obésité.

3. Composition comprenant un produit à base de protéines pour l'utilisation selon la revendication 2, la composition fournissant une dose qui est de 1,5 mg à 15 mg/kg de poids corporel/jour chez un mammifère et une dose humaine équivalente qui est de 5-100 mg/kg de poids corporel/jour, et comprenant un ou plusieurs excipients.

4. Produit à base de protéines pour l'utilisation selon les revendications 2 à 3, l'utilisation étant par administration orale à un mammifère et comprenant la réduction des triglycérides, du cholestérol total, du cholestérol à lipoprotéines de basse densité (LDL) et du poids corporel.

5. Produit à base de protéines pour l'utilisation selon les revendications 2 à 3, l'utilisation comprenant une réduction des graisses accumulées dans les adipocytes de mammifères.

6. Produit à base de protéines pour l'utilisation selon les revendications 2 à 3, l'utilisation comprenant une diminution de la masse de tissu adipeux.

7. Produit à base de protéines pour l'utilisation selon les revendications 2 à 3, l'utilisation comprenant une diminution de la synthèse de triglycérides et de cholestérol dans les cellules hépatiques de l'humain.

8. Produit à base de protéines pour l'utilisation selon les revendications 2 à 3, l'utilisation comprenant une augmentation des taux de cholestérol à lipoprotéines de haute densité (HDL) chez les mammifères.

9. Produit à base de protéines pour l'utilisation selon les revendications 2 à 3, l'utilisation comprenant le maintien du rapport LDL/HDL chez le mammifère quand administré à une dose de 1,5 mg à 15 mg/kg de poids corporel/jour.

10. Produit à base de protéines pour l'utilisation selon les revendications 2 à 3, l'utilisation étant par une administration à une dose humaine équivalente qui est de 5-100 mg/kg de poids corporel/jour.

11. Produit à base de protéines pour l'utilisation selon les revendications 2 à 3, l'utilisation étant par administration orale sous une forme solide ou liquide avec ou sans aucun excipient.
